(19) Europäisches Patentamt
European Patent Office
Office européen des brevets

(11) EP 2 279 768 A1

(12) EUROPEAN PATENT APPLICATION
published in accordance with Art. 153(4) EPC

(43) Date of publication:
**02.02.2011 Bulletin 2011/05**

(21) Application number: **09729362.5**

(22) Date of filing: **25.03.2009**

(51) Int Cl.:
***A61M 1/14*** *(2006.01)*

(86) International application number:
**PCT/JP2009/055968**

(87) International publication number:
**WO 2009/125674 (15.10.2009 Gazette 2009/42)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **09.04.2008 JP 2008101256**

(71) Applicant: **JMS Co., Ltd.**
**Hiroshima-shi, Hiroshima 730-8652 (JP)**

(72) Inventor: **IKEDA, Atushi**
**Hiroshima-shi**
**Hiroshima 730-8652 (JP)**

(74) Representative: **Röthinger, Rainer**
**c/o Wuesthoff & Wuesthoff**
**Patent- und Rechtsanwälte**
**Schweigerstrasse 2**
**D-81541 München (DE)**

# (54) HAEMODIALYSIS DEVICE

(57) Provided is a haemodialysis device in which the water removal rate can be rapidly and finely controlled so that the amount of blood can be suitably changed during haemodialysis, and in which there is an improvement in the control characteristics because the structure of the control means is simple and the operation is simplified. A haemodialysis device in which a time profile comprising target blood index values is preset as a target control line, and the dialysis conditions are controlled on the basis of blood index values measured or calculated at each control point over time in order to carry out haemodialysis, wherein the blood index value calculated taking account of the control delay at the control point one before a selected control point, the blood index value measured or calculated at that control point, and the target blood index value at the control point one after the selected control point are used to calculate the rate of water to be removed between the selected control point and the control point one after the selected control point, and water is removed between the two control points so that the target blood index value is reached.

FIG 1

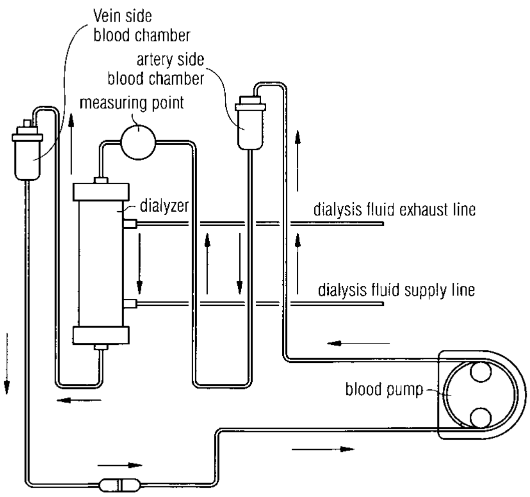


EP 2 279 768 A1

## Description

TECHNICAL FIELD

The present invention relates to a blood treatment apparatus, especially to a hemodialysis apparatus which can control water removal conditions, for example water removal speed so as to prevent excessive water removal and also lack of water removal in the contrary, which occur frequently during hemodialysis.

BACKGROUND ART

For treating patients with impaired kidney function, treatments by purifying blood by dialysis or filtration via semipermeable membrane have been provided conventionally. As for this apparatus, it is important to appropriately maintain the blood volume circulating in the patient body, to perform safe and effective blood purification. A rapid or excessive water removal will decrease excessively patient's blood circulating volume, and it may cause reduction of blood pressure, shock or the like. On the contrary, if the water removal is slow, it will take a long time for blood purification, and if sufficient water removal cannot be made, there is a fear that hypertension, heart failure or the like can occur.
Therefore, a hemodialysis apparatus performing water removal by monitoring patient's blood condition have been developed. For example, in Japanese Laid-Open Patent Publication No. 6-83723 (Patent reference 1,) an estimating apparatus which estimates the body fluid condition with a hematocrit meter, and a controlling apparatus which controls the blood pump or ultra pressure by the output of said estimating apparatus are described. Concerning this apparatus, it is convenient as the water removal is controlled directly according to the measured body fluid condition, but on the other hand, as the water removal is controlled directly by the measured value, in case the measuring means is not accurate or a trouble happens, it may cause a significant problem. Therefore, in such feed-forward control, generally, a separate line independent from the control line is disposed and a safety mechanism is loaded on said line. However, the apparatus becomes complicated when an independent line or safety mechanism is disposed and the operation becomes difficult. Furthermore, the cost of the apparatus will rise.
Therefore, a simple apparatus as described in Japanese Laid-Open Patent Application No. 9-149935 (Patent reference 2) was also developed. In other words, while monitoring the patient's blood condition, an alarm is ringed depending on the condition, and the water removal pump is stopped. However, this apparatus only recognizes if the water removal control is performed under the same control condition at the time of the initiation of dialysis, by comparing with the blood concentration measured before the initiation of dialysis, and it is not possible to perform adequate water removal to each patent. Furthermore, if the water removal is not performed according to the condition, the operator has to adjust each time the water removal volume or substitutive fluid volume. Thus, even though it was safe, it was complicated and the human cost was high. Moreover, as for said apparatus, a means for measuring the blood condition is disposed on the line at the vein fluid side of the blood cycle, the blood condition after having passed the blood treatment machine (dialyzer) is measured, thus it may not reflect the patient's direct blood condition.
To provide a blood treatment apparatus which have solved the problems mentioned above, that is, to provide a convenient apparatus at a low cost by making a structure wherein each patient's blood condition is monitored, enabling to perform blood treatment adequate to each patient chronologically, by not imposing much burden to the operator during its use, and by making the construction of the blood treatment apparatus simple, the present inventors provided a blood treatment apparatus, comprising a blood measuring means for measuring blood parameters; a working unit for performing blood treatment; and a controlling unit controlling the working unit to perform blood treatment under prescribed blood treatment condition, wherein the controlling unit prescribes the change of the blood treatment condition to the working unit, by setting a blood indication region determined beforehand against the patient blood indication level obtained with said blood measuring means, according to the chronological transition of said blood indication level in said blood indication region (Japanese Laid-Open Patent Application No. 11-22175: Patent reference 3).
Furthermore, the present inventors have improved said blood treatment apparatus (Japanese Laid-Open Patent Application No. 11-22175: Patent reference 3), and proposed a blood treatment apparatus (Japanese Laid-Open Patent Application No. 2001-540: Patent reference 4), wherein by monitoring each patient's blood condition, the condition of hemodialysis adequate to each patient chronologically, especially the water removal speed can be easily changed and defined. Said blood treatment apparatus of Patent reference 4 is a hemodialysis apparatus comprising at least: (A) a blood measuring means for measuring blood parameter, (B) a working unit for performing blood treatment; and (C) a controlling unit for controlling the working unit to perform blood treatment under prescribed blood treatment condition; wherein the hemodialysis apparatus has a mechanism for controlling the water removal speed, and the controlling unit (C) incorporates the blood indicating level obtained from the patients' samples by the blood measuring means (A), monitoring if it transits or not within the defined range the defined beforehand of blood indication level (hereinafter also referred to as defined range of blood indication level), and when said blood indication level being the target to control deviates from said range defined beforehand, the water removal speed of the working unit (B) can be changed at a speed rate defined beforehand.

As for the hemodialysis apparatus mentioned above, it was possible to manage reliably the blood indication level at each point during hemodialysis treatment, but because it was necessary to define the region of the target blood indication level at each point, the operation was complicated.

Also, since the target blood indication level was specified as a range, there was a problem that the mechanism for controlling the hemodialysis apparatus did not operate even when the blood indication level measured was barely within the target range, so far as the blood indication level was within the target range.

The inventor of the present invention further provided a hemodialysis apparatus comprising: a blood measuring unit which measures the blood parameters ; a working unit which performs blood treatment; and a controlling unit which controls the working unit so that blood treatment is performed in a prescribed condition, wherein the controlling unit transforms the blood parameters to a target blood indication level, and sets a chronological path comprising the target blood indication level as a target control line in advance, and further controls a water removing speed by utilizing the blood indication level at the previous controlling point adjacent to the chosen controlling point, one at the chosen controlling point, and one at the following controlling point adjacent to the chosen controlling point (Patent reference 5: WO03/004076A1 and Patent reference 6: US7,303,667 B2.)

[Patent reference 1] Japanese Patent H6-83723
[Patent reference 2] Japanese Laid-Open Patent Application H9-149935
[Patent reference 3] Japanese Laid-Open Patent Application H11-22175
[Patent reference 4] Japanese Laid-Open Patent Application H2001-540
[Patent reference 5] WIPO Patent Publication WO03/004076A1
[Patent reference 6] US7,303,667B

DISCLOSURE OF THE INVENTION

PROBLEMS TO BE SOLVED BY THE INVENTION

The object of this invention is to provide a hemodialysis apparatus having improved controlling characteristics with simple mechanism of controlling measures and easy operation of dialysis, so that it can realize speedy operation and accurate control of the dialysis, by taking into account the delay of the controlling point in the hemodialysis apparatuses disclosed in the patent references 5 and 6.

The hemodialysis apparatus of the present invention comprises a blood measuring unit which measures the blood parameters, the working unit which performs blood treatment; and the controlling unit which controls the working unit so that blood treatment is performed in a prescribed condition, wherein the controlling unit performs following four steps;

1) transforming the blood parameters measured by the measuring unit to a blood indication level intended,
2) setting a chronological path, comprising blood indication levels at every measuring points, is set as a target control line (hereafter, the measuring point is also referred as controlling point,)
3) calculating a water removing speed to be performed during a chosen controlling point n and a following controlling point (n+1) adjacent to the chosen controlling point, by utilizing the blood indication level at a controlling point (n-1); a previous controlling point adjacent to the chosen controlling point, one at the chosen controlling point n, and one at the controlling point (n+1), and controlling the water removing speed during the chosen controlling point n and the following controlling point (n+1), that is preformed at the working unit.
4) Using a blood indication level for the controlling point (n-1) taking into account the delay of the control.

The hemodialysis apparatus of the present invention can control the water removing speed accurately and stably by having the controlling unit as described above. That is, in the hemodialysis apparatus of the present invention, even when the controlling unit orders the working unit by a controlling order to start the water removing at the water removing speed A at the controlling point (n-1), the pervious controlling point adjacent to the chosen controlling point, and the water removing is actually started, the point of calculating an actual change in the blood indication level is not at the measuring point when the controlling order was ordered but at the point when the system reached to the measuring point again after passing dialyzer, vein side circuit and artery side circuit, starting from the measuring point, as shown in FIG. 1.

Thus, the present inventor found that in order for the controlling means to control the water removing speed accurately and stably, the change in the blood indication level during the control delay time, or the blood indication level taking into account the control delay time is essential as for the blood indication level at the controlling point (n-1) in the control of above described (3), instead of the blood indication level at the measuring point of the controlling point (n-1), as shown in FIG. 3, and reached to the hemodialysis apparatus of the present invention, where the control delay time is the time required to reach to the measuring point again after passing through dialyzer, vein side circuit, artery side blood chamber and artery side circuit, starting from the measuring point of controlling point (n-1).

The hemodialysis apparatus of the present invention preferably shall have the controlling unit which controls the hemodialysis operation according to following equation (a).

$$BV_0 \{-(\%\Delta BV_{n-1}' - \%\Delta BV_n) + (\%\Delta BV_n - \%\Delta BV_{n+1})\} + UFR_n \times T = UFR_{n+1} \times T \cdots\cdot (a)$$

wherein B V is the blood volume at the time of the initiation of dialysis; $\%\Delta BV_n$ is a ratio of the blood volume change at the selected measurement point (n); $\%\Delta BV_{n-1}'$ is a ratio of the blood volume change taking into account control delay at a previous controlling point (n-1), adjacent to the selected measurement point (n); $\%\Delta BV_{n+1}$ is a ratio of the blood volume change defined by the target control line at a next controlling point (n+1), adjacent to the selected controlling point(n); T is the elapsed time until the selected controlling point (n); $UFR_n$ is the water removal speed at the selected measurement point (n); $UFR_{n+1}$ is a calculated water removal speed defined at which the actual working unit carries out the water removal from the selected controlling point (n) to the controlling point (n+1), to attain the target ratio of the blood volume defined as a next controlling point (n+1).Here the apostrophe" ' " in $\% \Delta BV_{n-1}'$ means that the effect of the control delay time has been taken into account, instead of mere $\% \Delta BV_{n-1}$ value.

Above equation (a) is obtained as follows:

when each measurement point or controlling point is set as 1,2 · · · · · n-1, n, n+1, following equations are obtained.

The first controlling point and the second controlling point.

$$BV_0 [(100\% - \%\Delta BV_1) - (100\% - \%\Delta BV_2)] / T = PRR1 - UFR1 \cdots\cdots (1)$$

$$BV_0 (-\%\Delta BV_1 + \%\Delta BV_2) / T = PRR_1 - UFR_1 \cdots\cdots (2)$$

$$BV_0 (-\%\Delta BV_2 + \%\Delta BV_3) / T = PRR_2 - UFR_2 \cdots\cdots (3)$$

Here, $BV_0$ is BV level which is the primary blood volume at the time of the initiation of dialysis, $\%\Delta BV_1$ is the ratio of the blood volume change at the first measurement point or controlling point, $\%\Delta BV_2$ is ratio of the blood volume change at the second measurement or controlling point and T is the elapsed time of dialysis.

Moreover, $\%\Delta BV_3$ is the ratio of the blood volume change determined by the target control line at the third measurement or controlling point.

Similar relations are obtained at each arbitrary measurement or controlling points (n-1) and (n), as shown in the following relational equation.

$$BV_0 (-\%\Delta BV_{n-1} + \%\Delta BV_n) / T = PRR_{n-1} - UFR_{n-1} \cdots (4)$$

$$BV_0\,(-\%\Delta BV_n+\%\Delta BV_{n+1})\,/T=PRR_n-UFR_n\cdots\cdot \quad (5)$$

By subtracting the equation (3) from the equation (2), the following equation (6) is obtained.

$$BV_0\,\{(\%\Delta BV_1-\%\Delta BV_2)-(\%\Delta BV_2-\%\Delta BV_3)\}\,/T= PRR_1-PRR_2+UFR_2-UFR_1\cdots \quad (6)$$

When it is hypothecated there is no difference between $PRR_1$ and $PRR_2$, that is, when the distance between the controlling points is defined to be short so as the patient's PPR at each controlling point does not vary substantially, the first and second terms in the right side are deleted. Thus, the following equation (7) is obtained.

$$BV_0\,\{-(\%\Delta BV_1-\%\Delta BV_2)+(\%\Delta BV_2-\%\Delta BV_3)\}\,/T+UFR_1 =UFR_2\cdots\cdots \quad (7)$$

In the equation (7), $\%\Delta BV_3$ is the target level at the next controlling point, and is a level to which the blood indication level is to be brought close by carrying out the control. $UFR_2$ is the water removal speed to define so that the blood indication level of the next controlling point approaches the target level mentioned above. The above $BV_0$, $\%\Delta BV_1$, $\%\Delta BV_2$ and T are levels already known, and $UFR_1$ is also known as the water removal speed used from the first measurement point to the second controlling point. Therefore, if the target level $\%\Delta BV_3$ is specified, the water removal speed as a dialysis condition can be calculated with the equation (7). On the contrary, if the hemodialysis treatment is carried out with calculated water removal speed, the BV level at the next measurement point (controlling point) can be brought close to the target level, that is the target control line.

Describing the above equation (7) for arbitrary controlling point (n) and (n-1), following equation (8) is obtained.

$$BV_0\{-(\%\Delta BV_{n-1}-\%\Delta BV_n)+(\%\Delta BV_n-\%\Delta BV_{n+1})\}+UFR_n\times T=UFR_{n+1}\times T\cdots(8)$$

In the equation (8), $BV_0$ is the blood volume at the time of the initiation of dialysis; $\%\Delta BV_n$ is the blood volume change at an arbitrary selected measurement point (n); $\%\Delta BV_{n-1}$ is the ratio of the blood volume change at a previous measurement point adjacent to the selected measurement point (n); $\%\Delta BV_{n+1}$ is the ratio of the blood volume change at a controlling point (n+1), which is the next controlling point adjacent to the selected controlling point (n), where the feed forward control is brought; T is the measured time; $UFR_n$ is the water removal speed at the selected measurement point; $UFR_{n+1}$ is the water removal speed in order to reach to the target blood indication level during the measurement point (n) and the following measurement point (n+1), set against the working unit in making control, respectively.

In said equation (8), $BV_0$ is the blood volume at the time of the initiation of dialysis; $\%\Delta BV_n$ is the blood volume change at an arbitrary selected measurement point (n); $\%\Delta BV_{n-1}$ is the ratio of the blood volume change at the previous measurement point of an arbitrary selected measurement point (n); $\%\Delta BV_{n+1}$ is the ratio of the blood volume change at the controlling point (n+1), which is the next controlling point adjacent to an arbitrary selected controlling point (n), set for the feed forward control; T is the measured time; $UFR_n$ is the water removal speed at the selected measurement point (n) ; $UFR_{n+1}$ is the water removal speed in order to reach to the target blood indication level during the measurement point (n) and the following measurement point (n+1) adjacent to the controlling point (n), set against the working unit in making control, respectively.

However, the above equations (7) and (8) are valid under the condition that "every controlling points have equal PRR," where the condition has been assumed in deriving those equations. In order to satisfy the condition, it is important to reduce the interval ($\Delta T$) between the adjacent measuring (controlling) points so that PPR does not have substantial difference. Further, a control error may arise in this control method due to the error in $BV_0$ at the start of dialysis, delay of control, and to other factors. In actual control, the accuracy of the control is secured by newly selecting the water removing speed at each time point (measuring point). For example, by making the interval of each measuring points (controlling points) short and by selecting the water removing speed at each measuring point, the error generated will become practically negligible.

The above PRR is an abbreviation for Plasma Refilling Rate, and is defined as speed of the blood plasma refilling from the body to the blood vessel, and shows the patient's water removal ability at each point. The PPR is calculated with the following equation

$$PRR_n - UFR_n = \Delta BV_n / T$$

wherein $PRR_n$ is the Plasma Refilling Rate at an arbitrary selected measurement point (n), $UFR_n$ is the water removal speed at an arbitrary selected measurement point (n), $\Delta BC_n$ is the blood volume change at an arbitrary selected measurement point (n), T is the elapsed time until an arbitrary selected measurement point (n)

The derivation method of the ratio of the blood volume change taking into account the delay of control at the measurement point (n-1) ($\%\Delta BV_{n-1}$' in above equation (a)), is explained below based on FIG.2.

Multiple data of %ΔBV, for example sampling data in p1 to p6, were measured during the control period T1, which is the control period between the controlling point (n-1) and the controlling point n, except for the control delay period B1. Based on those sampling data and the linear least square method, a line B3 showing the chronological change of the ratio of the blood volume change can be obtained. By extending the line B3 at the same slope toward the controlling point (n-1), the blood indication level at the controlling point (n-1), $\%\Delta BV_{n-1}$' can be deduced, including the effect of the control delay. In FIG.1, the horizontal axis is the lapse time of hemodialysis operation, the vertical axis is taken as %ΔBV. Conventional methods used for processing measured data can be used as for the least square method. Arbitrary period within the control period T1 can be chosen as for the control delay period B1.

In the above equation (a), $BV_0$ (the blood volume at the time of the initiation of dialysis) is given by the sum of the blood volume circulating in the patient body and the blood volume circulating outside the patient body, and can be obtained, for example, by following equation (S-1) in below 1, or equation (S-2) in below 2.

1. The first method of calculating the initial blood volume ($BV_0$) by the equation (S-1) below is explained based on FIG.3.

At the beginning of the dialysis, only the blood circulation outside of the patient body is made without the water removing since the blood volume is unstable. If the circulation is continued until the blood volume stabilizes, a blood corresponding the increased volume (shown in the Figure as inflow from cells) of the blood volume circulating outside the patient body (space outside the patient body) is deemed to move from cells to veins, when cell turgor pressure is enough high and water overflows from cells and accumulated in the intercellular substance. Therefore, the initial blood volume ($BV_0$) for each patient can be determined based on below equation (S-1), from the increased blood volume circulating outside the patient body (space outside the patient body) and %ΔBV. After BV level has stabilized, the dialysis including the water removal is started.

The initial blood volume ($BV_0$)= [the increased blood volume circulating outside of the patient body (space out side the body)] / %ΔBV -(S-1)

2. The second method of calculating the initial blood volume ($BV_0$) by the equation (S-2) below is explained based on FIG.4.

Using a hemodialysis apparatus which controls dialysis condition based on the blood indication level, only the circulation to outside of patient body is made at the initiation of the dialysis and the circulation is continued until the BV level stabilizes. The dialysis including the water removal is started after the BV level has stabilized and at the same time as the start of the dialysis, a water removal is made with a determined removal speed (water removal speed A) and for a certain period ΔT (for the period that PRR does not change,) then the change of the BV level, $\Delta VB_1$ is calculated. Further, a water removal is made with a different removal speed (water removal speed B) for the same period ΔT, then the change of the BV level, $\Delta BV_2$ is calculated. The initial blood volume ($BV_0$) can be calculated using the BV levels $\Delta BV_1$, $\Delta BV_2$ and the water removal speeds A, B, based on below equation (S-2), specifically;

$$BV_0 = \{(\text{water removal speed A}) - (\text{water removal speed B})\} / (-\%\Delta BV_1 + \%\Delta BV_2) \times \Delta T \text{ ----------------(S-2)}$$

The above equation (S-2) can be derived as follows;
$\Delta BV / \Delta T = PRR\text{-}UFR - \Delta BC_1/\Delta T + \Delta BV_2/\Delta T$ = (water removal speed A) - (water removal speed B)

$$\Delta BV_1 = BV_0 \{(100\% - \%\Delta BV_1) - (100\% - \%\Delta BV_2)\}$$

$$\Delta BV_2 = BV_0 \times \%\Delta BV_2$$

$$BV_0 / \Delta T (-\%\Delta BV_1 + \%\Delta BV_2) = \text{(water removal speed A)} - \text{(water removal speed B)}$$

$$BV_0 / \Delta T = \{\text{(water removal speed A)} - \text{(water removal speed B)}\} / (-\%\Delta BV_1 + \%\Delta BV_2)$$

However, $BV_0$ (the initial blood volume) in the above equation (a) may also be determined by the known method other than above (S-1) or (S-2).

The blood parameters measured at the measuring unit and the blood indication level calculated based on the blood parameters at the control unit of the hemodialysis apparatus of the present invention are explained below.

1. The blood parameters measured at the measuring unit

BV level or hematocrit level (also abbreviated as Ht)
The hematocrit level is obtained by converting the optical transmittance of the blood of a patient, for example;

2. The blood indication level calculated based on the blood parameters at the control means

(1) ΔBV

It refers to the change in the above blood volume (BV level), and is the change in the blood volume per unit time. For example it can be calculated from Ht according to the following equation.

$$\Delta BV \text{ level} = \text{(Ht at the time of the initiation of dialysis)}/\text{(Ht at the time of measurement)} - 1$$

(2) %ΔBV

It is the ratio of the blood volume change, and is shown by the following equation.

$$\%\Delta BV = \Delta BV\ (\Delta BV \text{ level at the time of measurement}) / BV_0\ (\Delta BV \text{ level at the time of the initiation of dialysis}) \times 100$$

(3) BV%

It is calculated by dividing the BV level at the time of measurement by $BV_0$ (primary blood volume) which is the BV level at the time of the initiation of dialysis and is expressed in percentage. It is shown by the following equation.

$$BV\% = BV \text{ level at the time of measurement} / BV_0 \text{ which is BV level at the time of the initiation of dialysis (primary blood volume)} \times 100$$

EMBODIMENTS

The hemodyalisis apparatuses of the present invention include, for example, a hemodyalisis apparatus comprising

a blood measuring unit which measures the hematocrit level of a patient as the blood parameter,
a working unit which performs the blood processing, and
a controlling unit which calculates a ratio of the blood change rate %ΔBV to be targeted, based on the hematocrit level, also sets a chronological path comprising %ΔBV as target control line beforehand the start of the dialysis, and which controls the working unit based on the equation (a) described above.

The dialysis operation by the hemodialysis apparatus of the present embodiment is performed in the embodiment as illustrated in FIG. 5. That is, in the first half of the hemodialysis operation, using the target control line A' based on the above equation (a), the water removal is controlled rapidly in order to move the blood volume circulating in the body to the standard blood volume (BVst.) At the point (q point) when the blood volume circulating inside the body reached to the standard blood volume (BVst) through the water removal of the first half of the hemodialysis operation, the operation moves into the latter half of the hemodialysis operation. In the latter half of the dialysis operation, the water removal was performed maintaining the standard blood volume (BVst) according to the targeted control line D. In the water removal operation, the dialysis condition can be controlled more precisely by using the equation (a) which has taken into account the control delay time, than in the case where the equation (a) which has not taken into account the control delay time is used.

As for the $BV_0$ (the initial blood volume) in the equation (a), a value calculated by the above (S-1) method was used. The interruption line between the controlling point a and the controlling point (n-1) in FIG. 5 means that the indication of the controlling points were abbreviated between the controlling point a and the controlling point (n-1.) In the present embodiment, the dialysis performance was divided into two parts, the first half and the latter half, but a whole process of the dialysis may be performed using the equation (a.)

BREIF DESCRIPTION OF THE DRAWINGS

FIG.1 illustrates the configuration of the hemodialysis apparatus of the present invention.
FIG. 2 indicates the calculation method of $\%\Delta BV_{n-1}$' which has taken into account the control delay at the controlling point (n-1) in the hemodialysis apparatus of the present invention.
FIG. 3 indicates the calculation method (S-1) of the initial blood volume in the equation (a) which is used for the control of the hemodialysis apparatus of the present invention.

FIG.4 indicates the calculation method (S-2) of the initial blood volume in the equation (a) which is used for the control of the hemodialysis apparatus of the present invention.

FIG.5 illustrates the dialysis operation using the target control line in the hemodialysis apparatus of the present invention.

EXPLANATION OF NOTATIONS

A: The target control line based on the equation (a)
A': The target control line in the first half of the dialysis operation
D: The target control line in the latter half of the dialysis operation
B1: The control delay time
B2 : the line extended from the line B3 at the same slope to the controlling point (n-1)
B3 : The line calculated based on the least square method from the multiple data of $\%\Delta BV_n$ (p1 to p6), sampled during the period of control period T excluding the control delay period B1.
n: The selected controlling point
n-1: The previous controlling point adjacent to the controlling point n
n-2: The following controlling point adjacent to the controlling point n $\%\Delta BV_{n-1}$: the ratio of the blood volume change that has not taken into account the control delay at the controlling point n-1.
$\Delta BV_{n-1}'$: the ratio of the blood volume change that has taken into account the control delay at the controlling point n-1.
T1: the control period
T2: the control period

p1: the sampling data sampled during the control period T1 excluding the control delay period B1 (%ΔBV).
p2: the sampling data sampled during the control period T1 excluding the control delay period B1 (%ΔBV).
p3: the sampling data sampled during the control period T1 excluding the control delay period B1 (%ΔBV).
p4 : the sampling data sampled during the control period T1 excluding the control delay period B (%BV).
p5: the sampling data sampled during the control period T1 excluding the control delay period B1 (%ΔBV).
p6: the sampling data sampled during the control period T1 excluding the control delay period B1 (%ΔBV).
a: a controlling point
n-1: a controlling point
n+1: a controlling point
o : a controlling point
p: a controlling point
q: a controlling point
r: a controlling point
s: a controlling point
t: a controlling point
u: a controlling point
v: a controlling point
x: a controlling point
y: a controlling point

INDUSTRIAL APPLICABILITY

The present invention provides a hemodyalisis apparatus having excellent effects of following (1) to (4).

(1) The control mechanism is simple, and there is little risk of mistaken operation or out-of-control.
(2) As there is no need of difficult installation or unnecessary operation, the operator can easily operate without difficulty.
(3) As the control is carried out rapidly and finely, the blood volume can be transited fairly during the hemodialysis.
(4) By carrying out a control by considering the control delay, the control of dialysis conditions can be carried out more exactly.

## Claims

**1.** A hemodialysis apparatus, comprising:

a blood measuring unit which measures the blood parameters;
a working unit which performs a blood treatment; and
a controlling unit which controls the working unit so that the blood treatment is performed in a prescribed condition, wherein the controlling unit performs following four control actions;

(1) transforming the blood parameters measured by the blood measuring unit to a blood indication level targeted,
(2) setting a chronological path, comprising blood indication levels at every measuring points, as a target control line (hereafter, the measuring points are also referred as controlling points,)
(3) controlling a water removing speed that is performed at the working unit, during a chosen controlling point n and a following controlling point (n+1), adjacent to the chosen controlling point, by calculating the water removing speed to be performed during the chosen controlling point n and the following controlling point (n+1) by utilizing a blood indication level at a controlling point (n-1), a previous controlling point adjacent to the chosen controlling point, a blood indication level at the chosen controlling point n, and a blood indication level at the controlling point (n+1), and
(4) using a blood indication level that has taken into account a control delay as the blood indication level at the controlling point (n-1).

**2.** The hemodialysis apparatus of claim1, wherein further the controlling unit controls a hemodialysis operation according to following equation (a):

$$BV_0\{-(\%\Delta BV_{n-1}'-\%\Delta BV_n)+(\%\Delta BV_n-\%\Delta BV_{n+1})\} + UFR_n\times T=UFR_{n+1}\times T \qquad \text{(a)}$$

wherein $BV_0$ is a blood volume at a time of initiation of the hemodialysis operation;
$\%\Delta BV_n$ is a ratio of a blood volume change at the chosen controlling point (n); $\%\Delta BV_{n-1}'$ is a ratio of a blood volume change that has taken into account a control delay at the previous controlling point (n-1) adjacent to the chosen controlling point (n);
$\%\Delta BV_{n+1}$ is a ratio of a blood volume change defined by the target control line at the following controlling point (n+1), adjacent to the chosen controlling point(n); T is an elapsed time until the chosen controlling point (n);
$UFR_n$ is a measured water removal speed at the chosen controlling point (n);
$UFR_{n+1}$ is a calculated water removal speed which is required in carrying out the water removal from the chosen controlling point (n) to the following controlling point (n+1), in order to attain the ratio of the blood volume change defined by the targeted control line.

**3.** The hemodialysis apparatus of claim1 or claim2, wherein the ratio of the blood volume change at the controlling point (n-1), that has taken into account the control delay is determined as;
the controlling unit obtains a line indicating a change in the ratio of the blood volume change using the linear least square method based on a plurality of data on the ratios of the blood volume changes, sampled at a plurality of timing points during the control period between the controlling point (n-1) and the controlling point n excluding the control delay period, and then,
an extrapolation of the line to the controlling point (n-1) gives the ratio of the blood volume change in question.

FIG 1

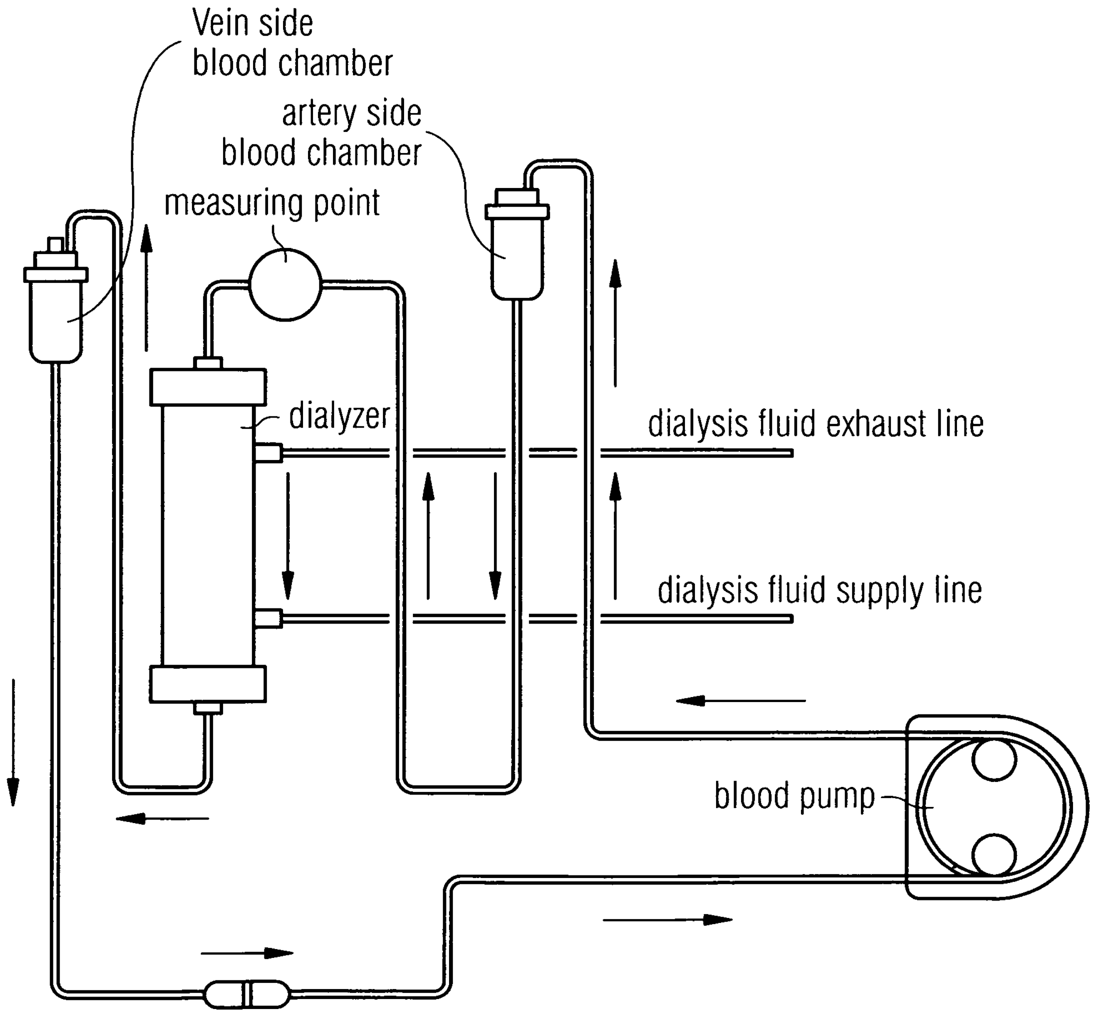
Vein side
blood chamber
artery side
blood chamber
measuring point
dialyzer
dialysis fluid exhaust line
dialysis fluid supply line
blood pump

FIG 2

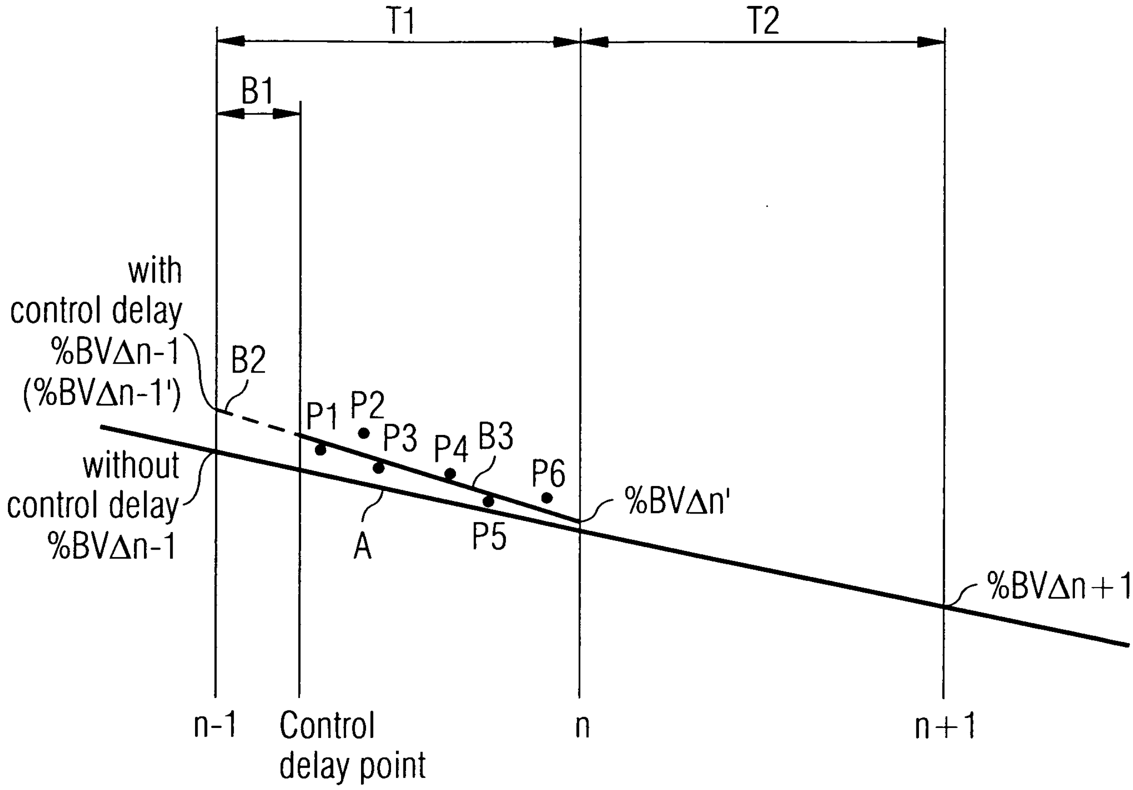
T1
T2
B1
with
control delay
%BVΔn-1
(%BVΔn-1')
B2
P1
P2
P3
P4
B3
P6
without
control delay
%BVΔn-1
A
P5
%BVΔn'
%BVΔn+1
n-1
Control
delay point
n
n+1

FIG 3

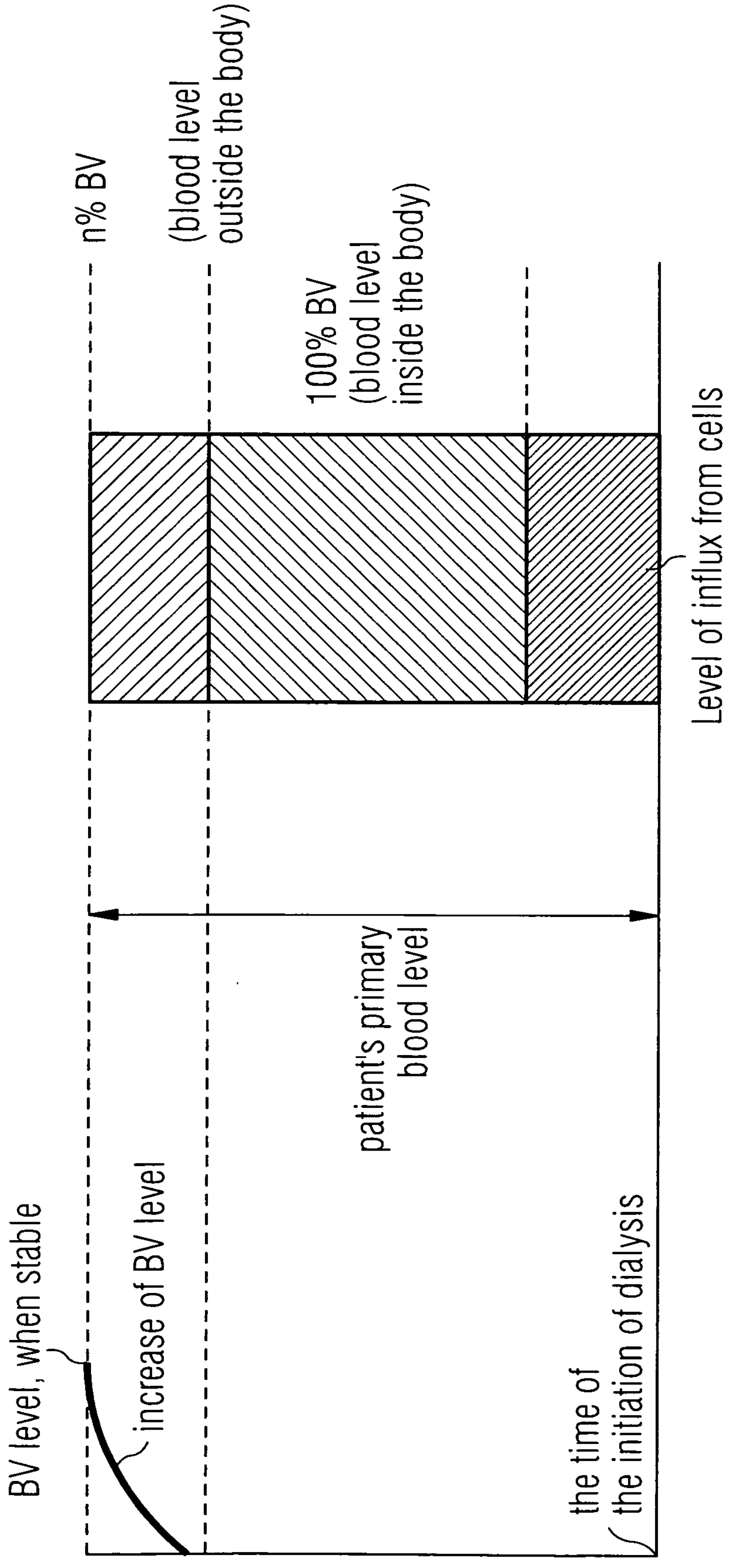
BV level, when stable
increase of BV level
n% BV
(blood level
outside the body)
100% BV
(blood level
inside the body)
patient's primary
blood level
Level of influx from cells
the time of
the initiation of dialysis

FIG 4

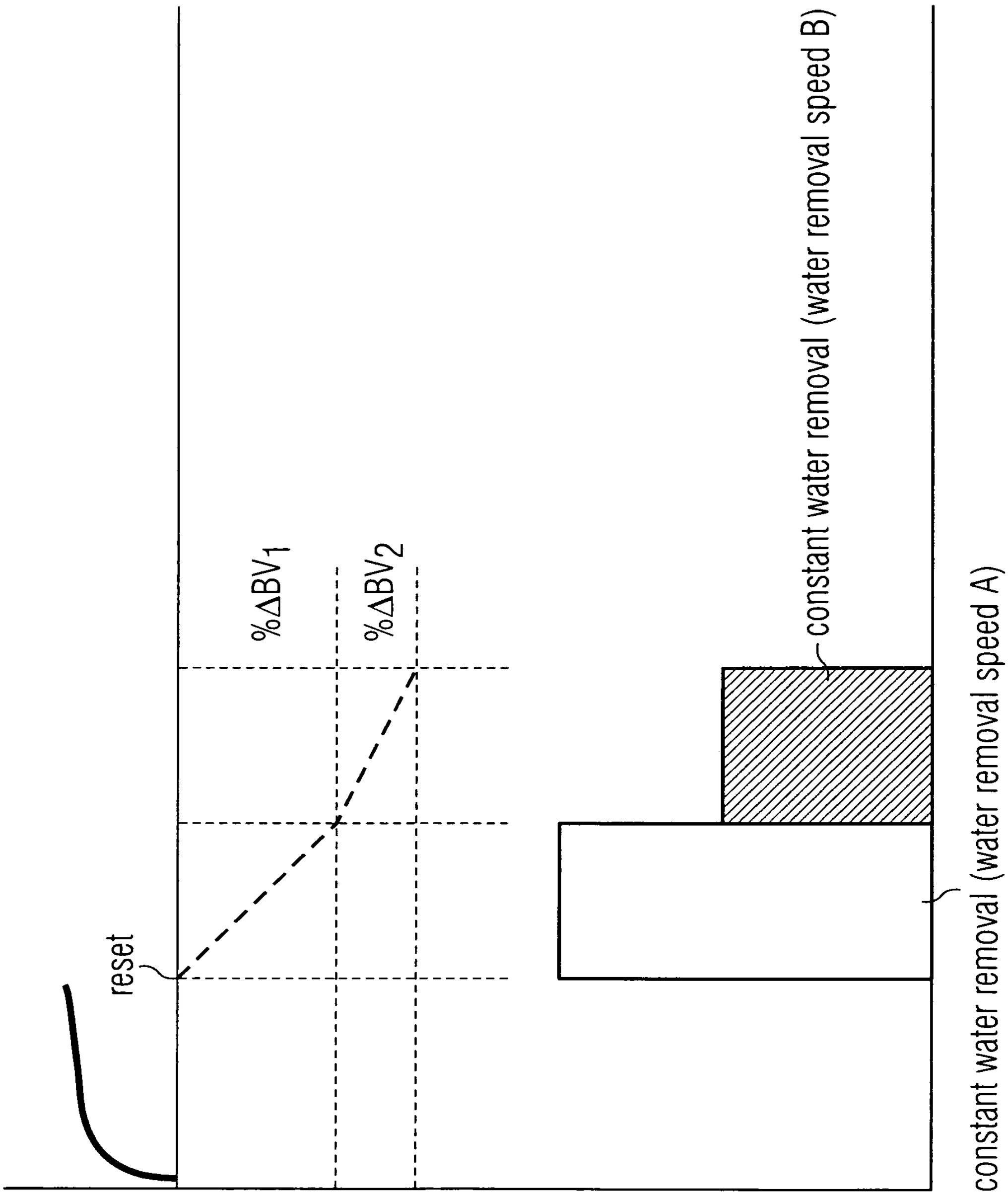
reset
%ΔBV1
%ΔBV2
constant water removal (water removal speed B)
constant water removal (water removal speed A)

FIG 5

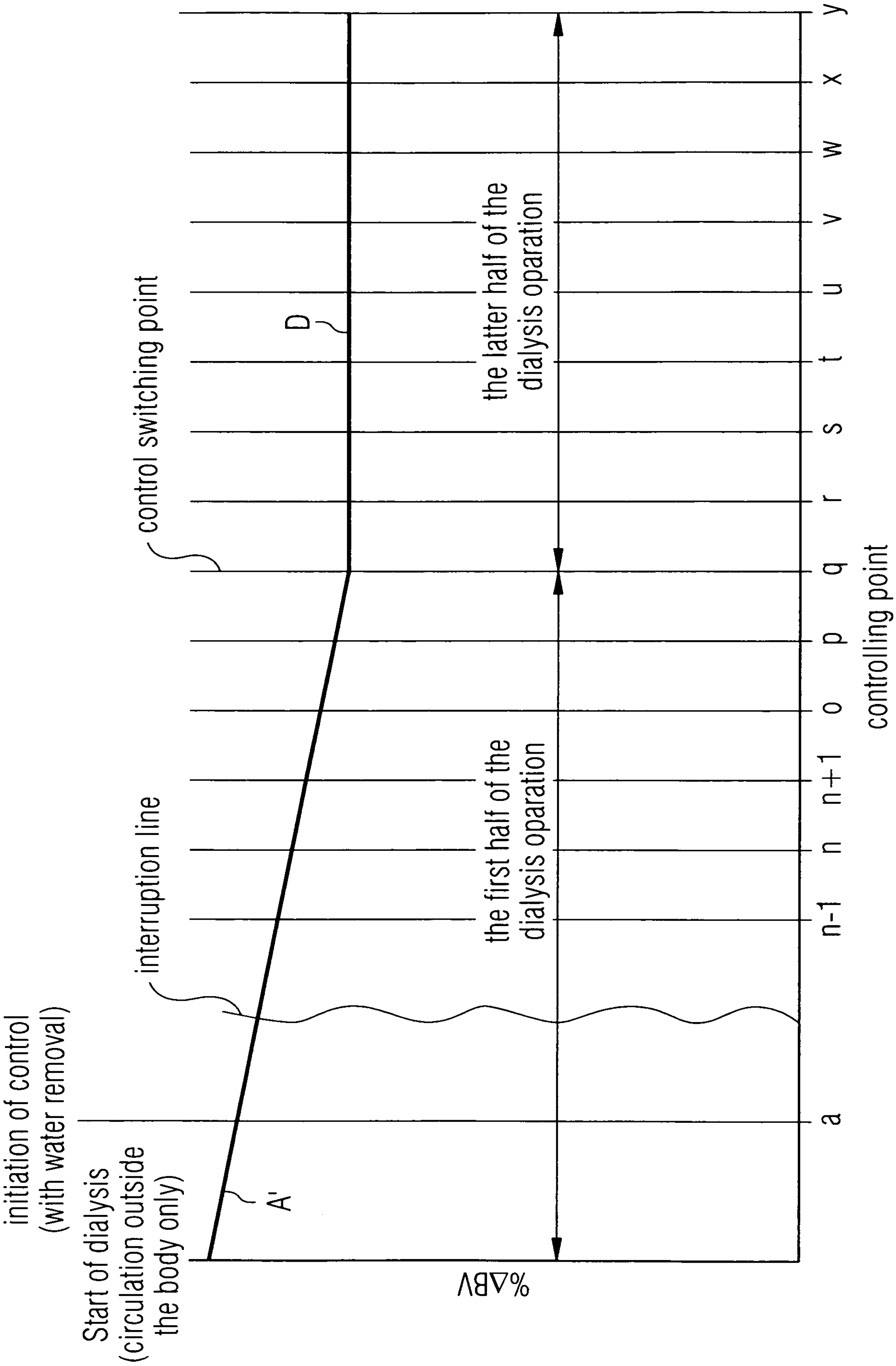
Start of dialysis (circulation outside the body only)
initiation of control (with water removal)
A'
interruption line
control switching point
D
%ΔBV
the first half of the dialysis oparation
the latter half of the dialysis oparation
a
n-1
n
n+1
o
p
q
r
s
t
u
v
w
x
y
controlling point

**INTERNATIONAL SEARCH REPORT**

International application No.
PCT/JP2009/055968

A. CLASSIFICATION OF SUBJECT MATTER
*A61M1/14*(2006.01)i

According to International Patent Classification (IPC) or to both national classification and IPC

B. FIELDS SEARCHED

Minimum documentation searched (classification system followed by classification symbols)
A61M1/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2009 |
| Kokai Jitsuyo Shinan Koho | 1971-2009 | Toroku Jitsuyo Shinan Koho | 1994-2009 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>A | WO 03/004076 A1 (JMS Co., Ltd.),<br>16 January, 2003 (16.01.03),<br>Pages 4 to 17<br>& JP 4225196 B & US 2004/0168988 A1<br>& US 2008/0105601 A1 & EP 1413324 A1 | 1,2<br>3 |
| A | WO 00/02604 A1 (FRESENIUS MEDICAL CARE DEUTSCHLAND GMBH),<br>20 November, 2000 (20.11.00),<br>Full text; all drawings<br>& JP 2002-520096 A & AU 5156999 A | 1-3 |
| A | JP 2001-540 A (JMS Co., Ltd.),<br>09 January, 2001 (09.01.01),<br>Full text; all drawings<br>(Family: none) | 1-3 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 19 June, 2009 (19.06.09) | 30 June, 2009 (30.06.09) |
| Name and mailing address of the ISA/<br>Japanese Patent Office<br>Facsimile No. | Authorized officer<br><br>Telephone No. |

Form PCT/ISA/210 (second sheet) (April 2007)

**INTERNATIONAL SEARCH REPORT**

International application No.
PCT/JP2009/055968

C (Continuation). DOCUMENTS CONSIDERED TO BE RELEVANT

| Category* | Citation of document, with indication, where appropriate, of the relevant passages | Relevant to claim No. |
|---|---|---|
| A | JP 2000-300 A (FRESENIUS MEDICAL CARE DEUTSCHLAND GMBH),<br>07 January, 2000 (07.01.00),<br>Full text; all drawings<br>& US 6602424 B1 & EP 960625 A2<br>& EP 1287838 A2 & EP 1927371 A2<br>& DE 19823811 C & DE 59903894 D<br>& DE 19823811 C1 | 1-3 |
| A | JP 11-221275 A (JMS Co., Ltd.),<br>17 August, 1999 (17.08.99),<br>Full text; all drawings<br>(Family: none) | 1-3 |
| A | US 5416027 A (Laboratoire Eugedia),<br>16 May, 1995 (16.05.95),<br>Full text; all drawings<br>& EP 551043 A1 & DE 69212132 C<br>& DE 69400094 C & FR 2685871 A<br>& FR 2687072 A | 1-3 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6083723 A
- JP 9149935 A
- JP 11022175 A
- JP 2001000540 A
- WO 03004076 A1
- US 7303667 B2
- JP H683723 B
- JP H9149935 B
- JP H1122175 B
- JP H2001540 B
- US 7303667 B